Europäisches Patentamt

European Patent Office

Office européen des brevets

(11) Publication number: **0 172 969**
**A2**

(12) # EUROPEAN PATENT APPLICATION

(21) Application number: **84307717.3**

(22) Date of filing: **08.11.84**

(51) Int. Cl.⁴: **G 01 N 33/48,** G 01 N 33/00

(30) Priority: **12.11.83 GB 8330268**

(43) Date of publication of application: **05.03.86**
**Bulletin 86/10**

(84) Designated Contracting States: **CH DE FR GB IT LI NL**

(71) Applicant: **Lion Laboratories Limited, Ty Verlon Industrial Estate, Barry South Glamorgan, CF6 3BE (GB)**

(72) Inventor: **Jones, Thomas Parry, 20, South Road, Sully Glamorgan (GB)**

(74) Representative: **Wynne-Jones, John Vaughan et al, Wynne-Jones, Lainé & James Morgan Arcade Chambers 33 St. Mary Street, Cardiff CF1 2AB (GB)**

(54) Discriminant analysis of gas constituents.

(57) Procedure and equipment for analysing gas samples, for example for detecting ethanol in human breath in the presence of other contaminants involves testing the sample by an electrochemical fuel cell, processing and analysing the fuel cell voltage to emphasise the differences between predetermined parameters obtained from different gas constituents, and so determining the analysis of the sample.

EP 0 172 969 A2

"Discriminant Analysis of Gas Constituents"


Electrochemical fuel cells are in widespread use throughout the United Kingdom and overseas for the measurement of breath alcohol.

Such fuel cells are highly specific for ethanol, when monitoring breath alcohol levels, but will respond to a variety of oxidisable organic species in industrial situations, and methanol and ethanol can cause interference in formaldehyde measurement. It would be unwise to use such a fuel cell based instrument, for the measurement of formaldehyde, where high levels of ethanol or methanol are present. The measurement of ethanol can be complicated, in a similar manner, by the presence of methanol or other oxidisable organics.

With the increased use of evidential breath instruments to replace blood or urine alcohol analysis, it is important that the breath alcohol reading presented to count is as unequivocal as that obtained from blood analysis, using gas chromatography or the ADH enzygmatic method.

It is generally accepted that the majority of breath alcohol instruments such as those based on infra-red, colorimetry or electrochemical principle, are quite adequate for measuring ethyl alcohol when it is the only volatile constituent present. However, when there are other breath volatiles present breath analysis is then open to criticism.

Although breath instruments have been developed based on gas chromatography the technique is not widely used mainly because, with the accompanying gas cylinders, it is not ideal for routine use by trained police officers at police stations.

Breath alcohol instruments based on electrochemical fuel cell sensors have some inherent specificity, as they do not respond to breath acetone or derivatives of organic hydrocarbons which are often encountered in industrial working environments. It has now been discovered that the specificity can be further enhanced by analysis of the shape of the response time curve from the sensor using a microprocessor, as the shape of the curve is a function of the gas species being oxidised at the fuel cell electrode. For instance, possible breath contaminants, i.e. aldehydes and alcohols such as methanol and propanol can be detected, even when present at levels below 10 mg %.

Accordingly it is an object of the invention to provide an improved procedure and equipment for identifying or detecting one or more from a plurality of constituents of a gas sample, in a simple and rapid manner.

Broadly stated from one aspect the invention consists in a method of detecting the presence of one or more of a plurality of constituents in a gas sample, using an electrochemical fuel cell, in which the gas sample is introduced into the cell, and the cell output voltage is sensed or recorded continuously or at a predetermined instant, or at intervals over a period of time, and the sensed values are processed or analysed to determine one or more of the following features:-

(a) Peak Time (the period for the cell output voltage to reach a peak),

(b) The integral of cell output voltage over a selected period (the "area under the curve"),

(c) The ratio of the area under the output curve during decay to the area under the curve over the whole test period,

(d) The mean normalised value of cell output voltage,

(e) The differential of the cell voltage as a function of time,

(f) The whole "shape" of the voltage curve by digital memory techniques.

In one method of carrying out the invention a standard gas sample containing a main constituent to be tested, is first admitted to the cell and one or more of the specified features are generated and recorded, and a test sample is then admitted to the cell, containing the aforesaid main constituent and possibly others unknown, and the features obtained from the cell on the second test are compared with those obtained from the first test.

The recorded "features" may be put to various different uses, but in many cases it is of advantage that the differences between the two features or sets are evaluated, and if they depart from a specified value or exceed a predetermined limit a further output signal is produced or inhibited.

When applying the invention to alcohol breath testing for legal purposes to determine whether a vehicle driver is within legal alcohol limits, the detection methods may also be used to provide automatic controls on the operation of the breath testing process. For example, if the test indicates that a contaminant is present at a specified level which is known to distort the proper output reading from the instrument, then the discriminator may either indicate or signal that the test is invalid or may operate an automatic inhibit device which renders the output of the test equipment inoperative.

In some industrial applications where preliminary tests on samples show large deviations for different constituents and contaminants the differential values obtained at the optimum points of the tests may be so large or clearly defined that a number of constituents may be identified, possibly on a quantative basis.

Thus in a preferred process according to the invention when applied to alcohol breath testing the features generated from a breath sample are compared with the recorded features from a standard alcohol breath sample applied previously to the instrument, and the difference is applied to an evaluator, and if above a specified limit is then caused to operate a warning signal or device which inhibits the operation of test or indicates that the test is invalid.

In one particular preferred procedure a sample of gas containing the main constituent to be tested, with one or more suspected contaminants, is first applied to the fuel cell and the derived features compared with the features obtained from the main constituent alone or with another contaminant, and optimum points of difference are selected, and when the gas test sample is applied to the cell the resulting features are compared with the said standards at the selected optimum points.

In any case the derivation of the features, and the comparison with standard features is preferably performed by an electronic computer or microprocessor, using an appropriate pre-arranged computer programme.

The invention also consists in apparatus for performing any of the procedures referred to above comprising an electrochemical fuel cell, means for delivering a gas sample thereto, and a computer or microprocessor arranged to receive the voltage output from the cell, and programmed to produce or derive one or more of the features specified, and optionally to compare a feature with a corresponding feature from a previous sample.

The invention may be performed in various ways and some details will now be described by way of example with reference to the accompanying drawings, in which:-

Figure 1 is a diagram showing a typical curve representing fuel cell output voltage over a period of time after being exposed to a gas sample,

Figure 2 is a diagram illustrating a typical electrochemical fuel cell, as used in the invention,

Figure 3 is another diagram illustrating the main components of a gas sampling and testing instrument, including a mini-computer for use in performing the invention,

Figure 4 is a diagram showing three curves representing the cell output voltage for methanol, ethanol and formaldehyde,

Figure 5 is a diagram illustrating the different voltages between different pairs of such gases,

Figure 6 is a frequency histogram showing the values obtained for "peak time" from the three gas examples,

Figure 7 is a frequency histogram for the first polynomial coefficients $t$ .

Figure 8 is a frequency histogram for the second polynomial co-efficient $t^2$,

Figure 9 is a cluster diagram obtained by reclassifying the data derived from a triple gas sample,

Figure 10 is a cluster diagram derived from this data reclassified by a step wise analysis, and

Figure 11 is a diagram illustrating the group centroids for comparison with previous analyses.

Some information about the presence or absence of interferants can be obtained by measuring

JWJ/MMD

the time taken for the cell output voltage to "peak". If the peak time is considerably longer or shorter than expected, or if the decay back to zero is very much slower, the presence of interferring gases or oxidisable vapours is to be suspected.

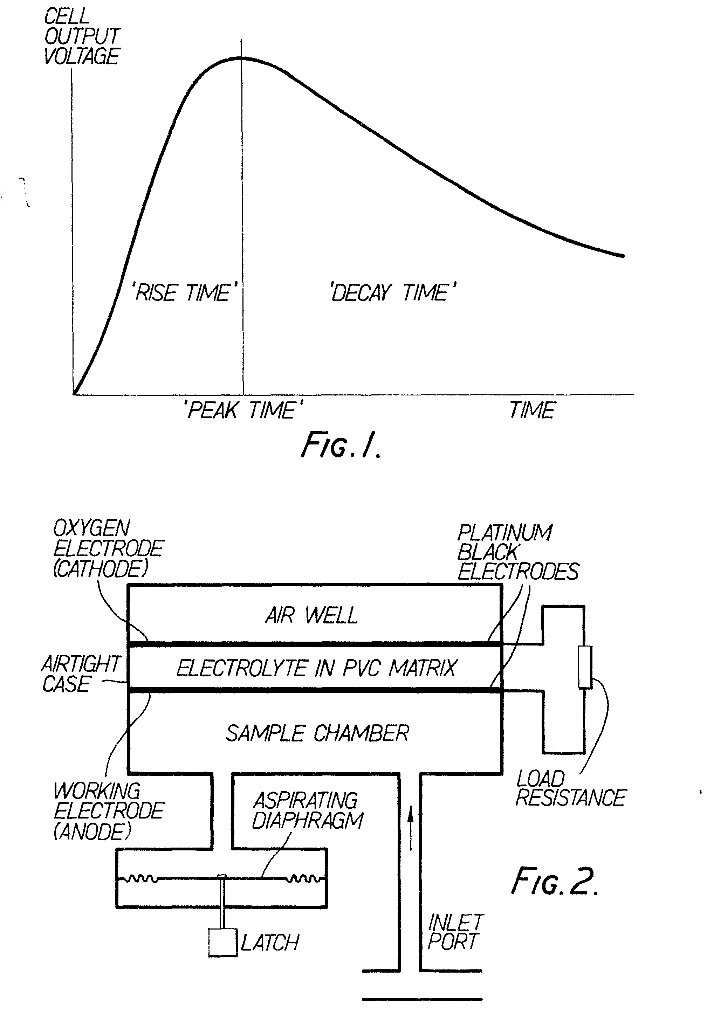

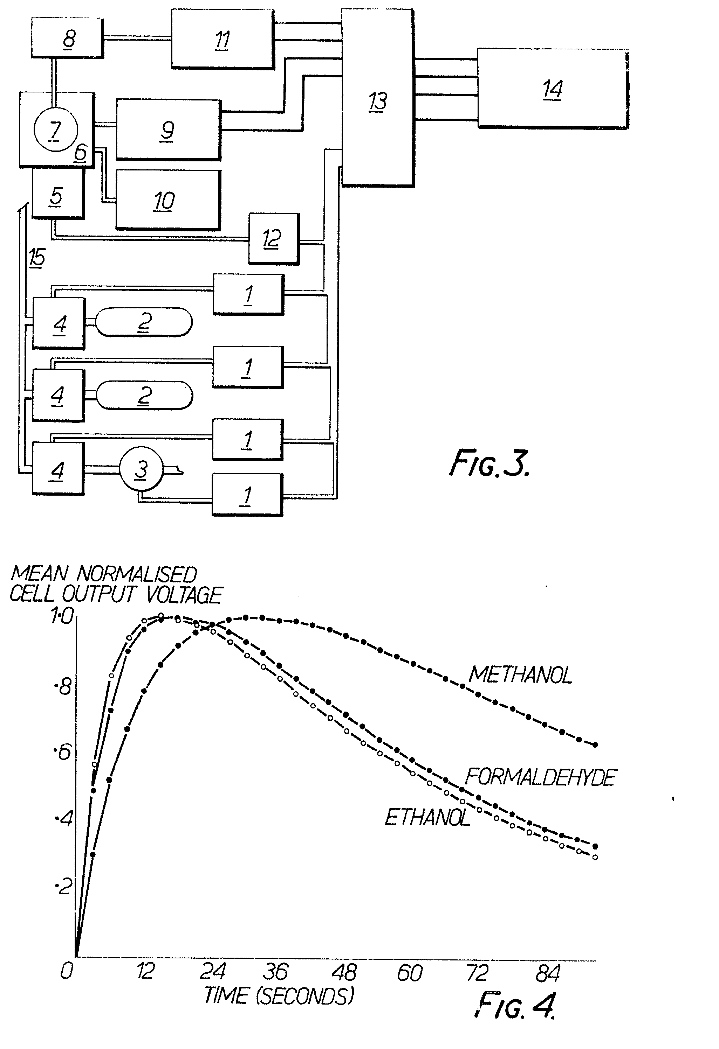

The structure of the fuel cell used in these tests is shown in Figure 2. The cell consists of two platinum black electrodes on gold carriers supported by a porous p.v.c. disc containing an immobilised acid electrolyte. When a sample is introduced into the space above the working electrode the oxidisable substance is absorbed and spontaneously oxidised to produce an electron flow. The electric current flowing from the working to oxygen electrode causes a potential change across an external load. The cell is connected to a commutating auto zero amplifier configured in a differential mode. The amplifier output is connected via an eight bit analogue to digital convertor (ADC) designed into an autoranging circuit with a sensitivity of 1 mV in 1 volt, operating under software control. The output from this device is connected to the data bus of a 32K Commodore Pet Minicomputer (Figure 3).

The fuel cell is maintained at a constant temperature of nominally $52^{\circ}C \pm 0.5^{\circ}C$. The temperature is constantly monitored using an electronic thermometer circuit interfaced to the minicomputer.

Samples to be analysed are drawn into the fuel cell, from a gas flow line, using a solenoid operated aspirating system. The gas flow is controlled by the computer using solid state relays and solenoid valves. Between samples and prior to each sampling sequence the common gas flow line is blown clear by a 30 second air flow from a computer controlled pump.

Data was collected in three experimental series. Each series consisted of fifty alternate samples taken at 50 minute intervals, of two of the three substances investigated, i.e. series one consisted of alternate samples of formaldehyde vapour (26 ppm) and ethanol (115 ppm), series two of methanol (175 ppm) and ethanol (115 ppm) and series three of methanol (175 ppm) and formaldehyde (26 ppm). The samples were arranged alternately to reduce the effect of any drop in sensitivity occurring during one sample series, while the duplication of samples in different series allowed the effect of any decreased sensitivity to be determined.

The cell output voltage rises to a peak much more rapidly than it decays to zero. Voltage samples of the cell output waveform were therefore taken at different rates during the rise and decay periods. The digitised data was stored in random access memory, data sampling being truncated 150 seconds after the cell peak output had been detected, by a software peak detector routine. The peak cell output voltage and the time, after injection of the sample, at which the peak value occurred (Peak Time) were also stored.

The data processing capabilities of the microcomputer were used to carry out a number of transformations of the stored date, after normalising to the peak value. The voltage output waveform from a fuel cell operated in the mode described is of the form

$$V = \frac{E_{max}}{(k_2/k_1) - 1} (e^{-k_1 t} - e^{-k_2 t})$$

The constants $K_1$ and $K_2$ can only be determined experimentally and cannot be derived from the shape of the cell output waveform. Two other equations were therefore used to try and derive a constant characteristic of the individual species.

The first of these equations

$$V_o = V_{max} e^{-kt^x}$$

was used to describe the decay portion of the cell output waveform. The values of k and x were determined.

The second equation was an eleventh order polynominal

$$V_o = a + b_1 t + B_2 t^2 \ldots b_{11} t^{11}$$

The constants a, and $b_1$ to $b_{11}$ were calculated using a modification of a least squares routine. This was adapted for operation with the minicomputer, from a
programme by Shapland.

The integral of the cell voltage output was
calculated using Simpson Rule and the mean normalised
value of the cell output voltage determined. The ratio
of the area under the output curve, during the decay
period, to that of the area under the curve for the
total sampling period was calculated.

Thirty two samples of the normalised cell
output voltage waveform taken at three second intervals,
were used to calculate the Discrete Fourier Transform
(D.F.T) using a subroutine available on the D.E.C.
VAX 11/780 (Nag Fortran Library, CO6EAF, (NAG FLIB:
1813/O, Mk8: 13 January, 1981). This computer was
also used to run the S.P.S.S. discriminant analysis
subroutines, used for data analysis (Statistical Package
for Social Scientists).

RESULTS AND DISCUSSION:

For the three species investigated a significant
decrease in cell peak output voltage (>0.01 level) was
found when comparing the means of two sampling series.
For instance the peak cell output for formaldehyde in
series one was 163 while in series two this dropped to
a value of 142. A significant temperature difference
(>0.01 level) was found between the two formaldehyde
sample series. This rise in temperature from a mean
value of $51.28^{\pm} 0.5^{\circ}C$ to $51.5^{\pm}0.5^{\circ}C$ would however be
expected to give an increase in peak cell output.

The decrease in output can be attributed to a reduction in cell sensitivity which occurs when large numbers of successive samples are taken.

The change in peak output was reflected by a drop in the mean total integral for all three species but other measured parameters i.e. $K, x$, the constants $a$, and $b_1$ to $b_{11}$, and the 'mean' cell output showed no significant change between sample series. Similarly the Fourier transform coefficients and time series values showed no significant alteration. This indicates that although the sensitivity of the cell may have changed, the shape of the output waveform remains constant.

In contrast there were marked differences between individual species within each sample series. The coefficients of the polynominal approximation $a$, and $b_1$ to $b_{11}$ were all significantly different at the 0.005 level.

The first eight Fourier transform coefficients (DC to 2.33 Hz) differed significantly for the three different species (>0.01 level). The remaining coefficients were significantly different for the methanol/ethanol samples (>0.005) and the coefficients for $f=2.67$ to 4.66 Hz for the methanol/formaldehyde samples (>0.01). The most significant differences, as determined using the 'Student's t' test, occurred for the first eight D.F.T. Coefficients (DC-2.33 Hz).

In general the three series of experiments indicated that while successive sampling caused a reduction in cell peak output this did not have any significant effect on the shape of the cell voltage output waveform.

Pooling the time voltage data for each species gave the results shown in Figure 4. This mean cell output voltage is shown plotted against time. The most marked difference in the shape of the cell output waveforms is between that for methanol and ethanol, or methanol and formaldehyde samples.

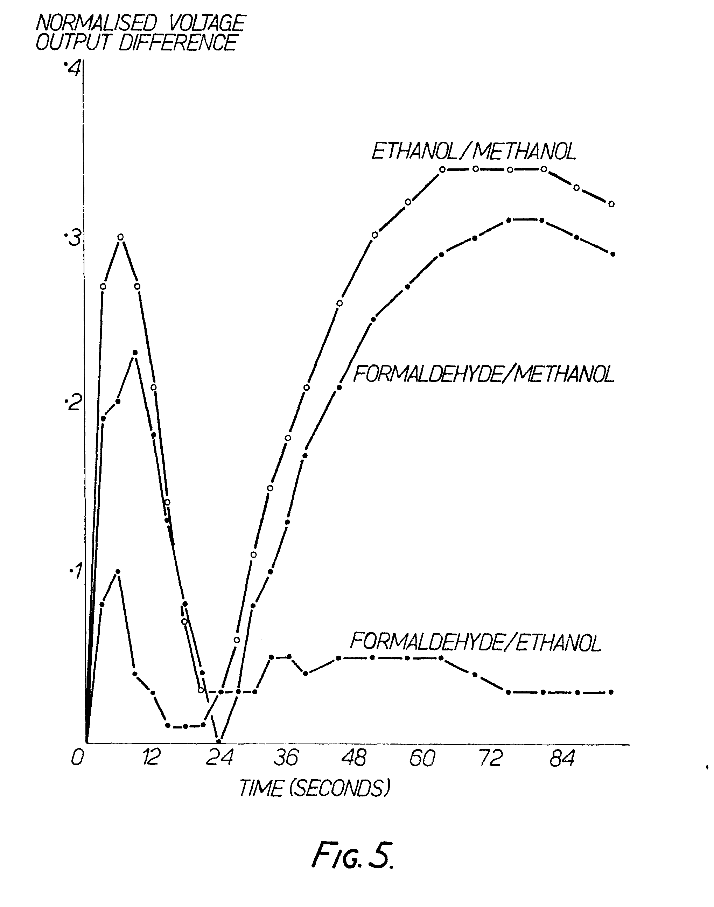

For methanol samples the peak is reached much more slowly (28.18 seconds) than for ethanol (13.12 seconds) or formaldehyde (15.34 seconds) and the cell output drops to only 0.6344 of the peak after 93 seconds as opposed to 0.3049 for ethanol or 0.3393 for formaldehyde. The difference between the cell output for methanol samples and those for the other two species indicate that the identification of methanol, as a single component injected into the cell can be accomplished by using one or more of the cell output voltage samples. Figure 5 shows the unsigned difference between the three mean voltage waveforms given in Figure 4, plotted against time. If a single sample point is to be used to identify one of the three compounds this can best be accomplished by taking a voltage measurement six seconds after injection of the sample. The variation of the data at this time is such that the three species form distinct groups. At all other times during the sampling period there is some merging of two of the three groups which makes the unique identification of ethanol or methanol impractical. The discrimination between methanol and either of the other two species is best conducted at a time 75 seconds after injection of the sample into the fuel cell.

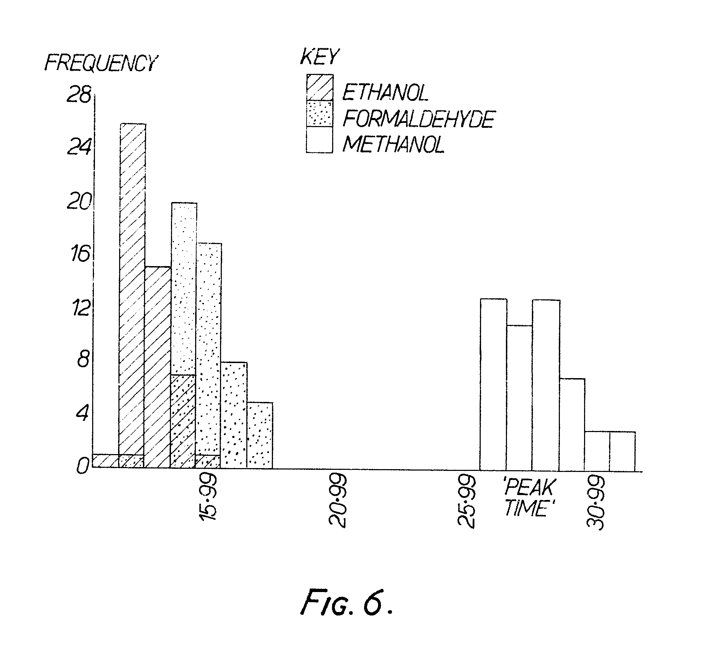

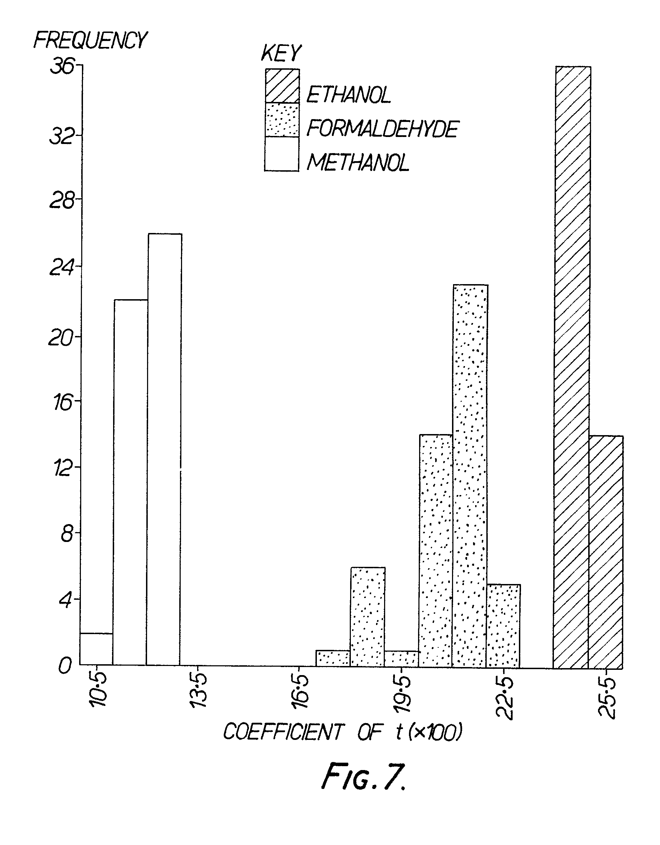

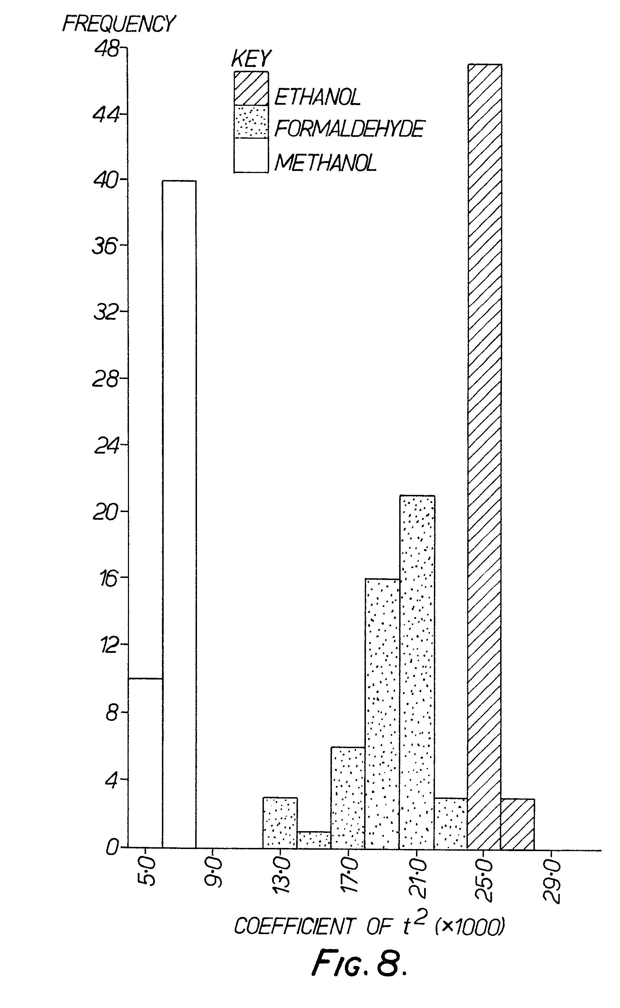

In general single variable discrimination between the three species is made difficult by the variability of the data. Figure 6 is a frequency histogram for the values obtained for 'peak time'. Methanol forms a discrete group on the 'peak time' axis but the other two groups, while having significantly different means (15.34, 13.12 seconds) are not distinct. The only single variables found which completely discriminate between the three species are the first two polynomial coefficients, i.e. for $t$ and $t^2$. The frequency histograms for these two variables are given in figures 7 and 8, where three distinct data groups can be seen.

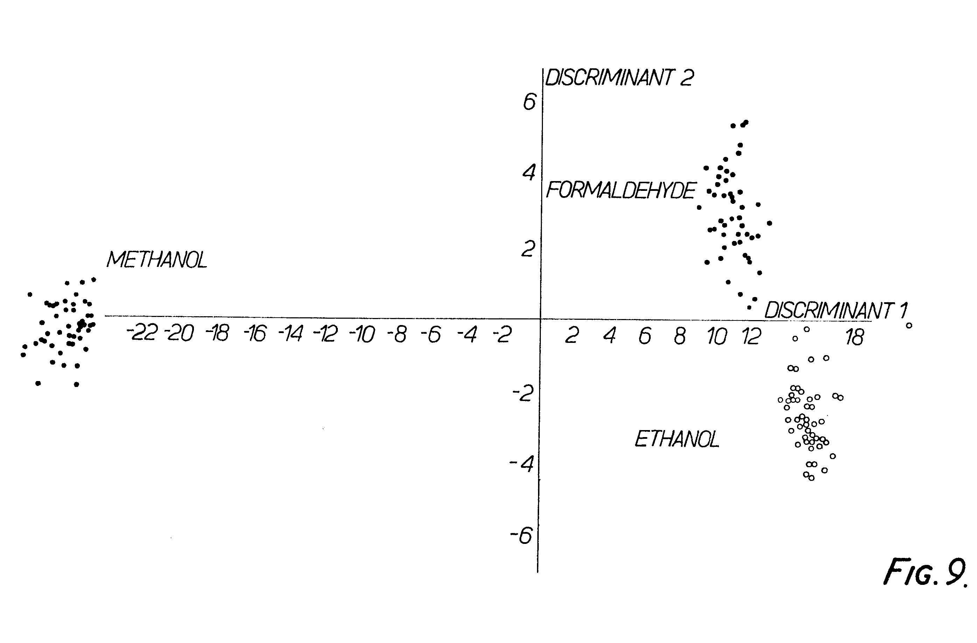

Multivariate discriminant analysis is used for the classification of sample data into discrete groups. In a two group analysis the data can be separated in a single dimension.  For the analysis of n groups then n-1 dimensions are required. Two dimensional discriminant analysis is therefore necessary to differentiate between the three species investigated. Subprogram 'Discriminant' available with S.P.S.S. allows the calculation of discriminant functions, using stepwise analysis to find those variables which give optimum separation. A number of different analytical methods, i.e. RAO, WILKS, MINRESID, MAXMINF and MAHAL are available. The coefficients derived from the D.F.T. were used as input to discriminant analyses using the methods MINRESID, MAHAL, WILKS, and RAO to determine which gave the greatest separation between the two most similar data sets (formaldehyde and ethanol). (MAXMINF and MAHAL will give the same results if the two groups are the same size). The group centroids and the separation between groups were the same for each case. Complete separation was obtained between the ethanol and formaldehyde data sets.  As all four methods gave the same results subsequent discriminant analyses were carried out using the method 'MAHAL', specifically chosen to maximise the distance between the two closest groups. Figure 9 is a cluster diagram obtained by reclassifying the D.F.T. data according to the discriminant equations derived from a three group analysis

The group centroids obtained from discriminant analyses using a number of different combinations of the investigated variables are shown in Figure In general the greater the number of variables added the greater the separation.

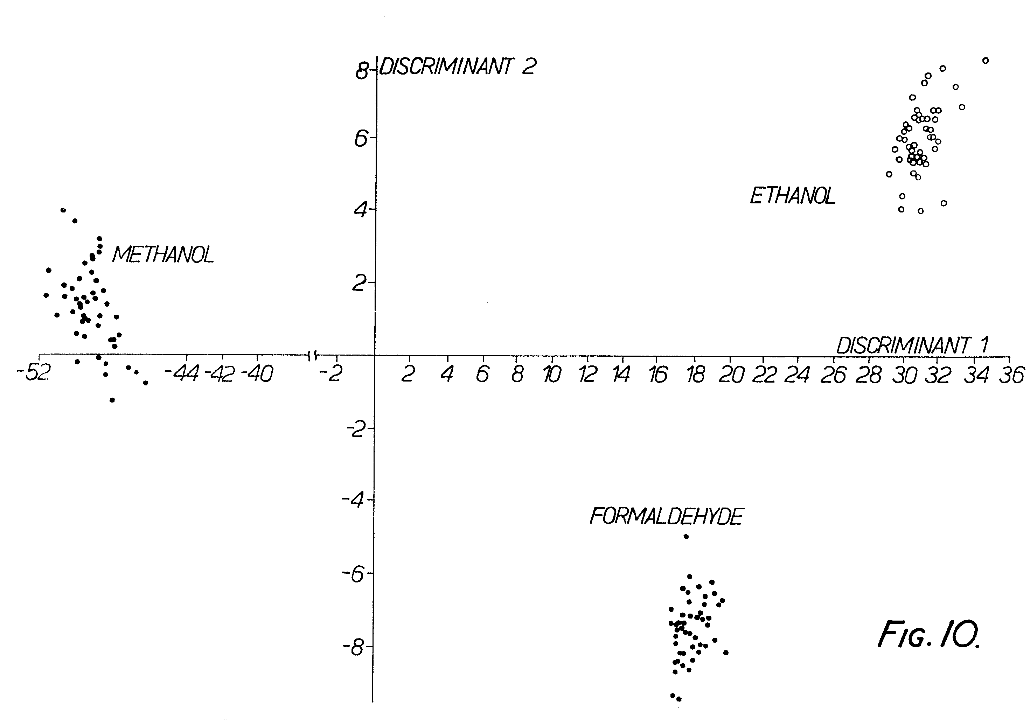

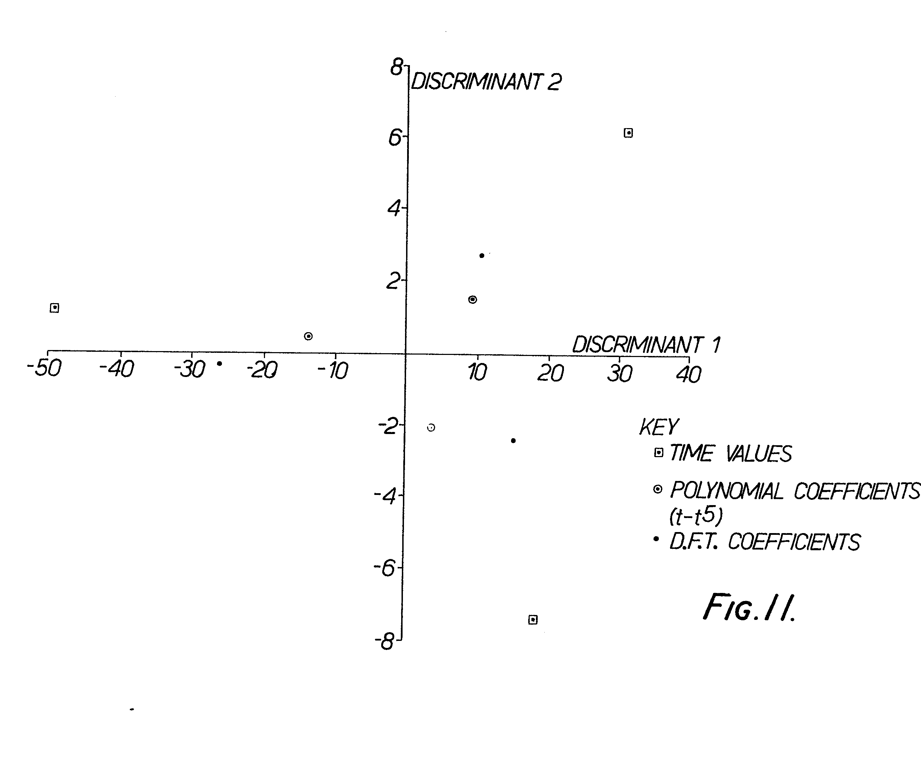

By far the greatest discrimination between the groups is obtained using the time/voltage output measurements as input to the discriminant analysis routine. Figure 10 shows a cluster diagram for this data reclassified according to the results of a stepwise analysis. For comparison with previous analyses the group centroids are shown in Figure 11. The separation between the methanol group and either of the other two groups is greater than that between ethanol and formaldehyde.

Identification of methanol in the presence of formaldehyde could be useful in industrial situations, where the two are commonly found together. The identification of methanol in the presence of ethanol could be useful in medical situations, where drunkenness could be attributed to either, but the former is poisonous.

TO SUMMARISE:

Discrimination between the three species can best be effected by using the sampled voltage output waveform from the fuel cell. The separation between the three groups is such that it is possible, using multivariate linear regression techniques, to achieve a semiquantitative method of measuring methanol concentration in the presence of formaldehyde or ethanol.

Due to the variability of the data a single normalised output voltage measurement is unlikely to provide sufficient discrimination for identification of any of the three species.

Transformations of the data to yield polynomial or fourier transform coefficients is complex, and although the former (for $t$ and $t^2$) can be used as diagnostic indicators for the three compounds, the simpler direct voltage measurement approach to their identification is more effective.

In Figure 3 the elements are:-

1. Solid State Relays.
2. Gas Samples.
3. Air Pump.
4. Solenoid Valves.
5. Sampling Solenoid.
6. Oven.
7. Fuel Cell.
8. C.A.Z. Amplifier.
9. Thermometer.
10. Temperature Controller.
11. Autoranging Circuitry.
12. Solenoid Control.
13. Digital Interface.
14. Minicomputer.
15. Gas Line.

CLAIMS

1. A method of detecting the presence of one or more of a plurality of possible constituents in a gas sample, using an electrochemical fuel cell (7), in which the gas sample is introduced into the cell, and the cell output voltage is sensed or recorded, and the sensed value or values are processed or analysed to emphasise any differences due to the presence of one or more selected constituents.

2. A method according to Claim 1, in which the cell voltage is analysed to determine the period for the cell output voltage to reach a peak.

3. A method according to Claim 1, in which the cell output voltage is processed to provide the integral of cell output voltage over a selected period.

4. A method according to Claim 3, in which the cell output voltage is processed to produce the ratio of the area under the voltage-time output curve during decay to the area under the curve over the whole test period.

5.  A method according to Claim 1, in which the cell output voltage is processed to provide the mean normalised value of cell output voltage.

6.  A method according to Claim 1, in which the cell output voltage is processed to provide the differential of the cell voltage as a function of time.

7.  A method according to Claim 1, in which the cell output voltage is processed and analysed to determine the whole shape of the voltage-time output curve by digital memory techniques.

8.  A method according to Claim 1, in which the cell output voltage is sensed on a predetermined time basis, either continuously or at intervals, or at a selected instant.

9.  A method according to Claim 1, in which a standard gas sample containing one constituent to be tested is first admitted to the cell and one or more of the selected parameters are generated and recorded, and a test sample is then admitted to the cell, containing unknown constituents, and the parameters obtained from the cell on the second test are compared with those obtained from the first test.

10. A method according to Claim 9, in which the differences between the two parameters are evaluated, and if above a specified value a further output signal is produced or inhibited.

11. A method according to any of Claims 1 to 10, forming part of an alcohol breath test, in which the parameters generated from a breath sample are compared with the recorded parameters from a standard alcohol breath sample applied previously to the instrument, and the difference is applied to an evaluator, and if above a specified limit is then caused to operate a warning signal or device which inhibits the operation of the test or indicates that the test is invalid.

12. A method according to any of the preceding claims, in which a sample of gas containing the main constituent to be tested, with one or more suspected contaminants, is first applied to the fuel cell and the derived parameters are compared with the features obtained from the main constituent alone or with another contaminant, and optimum points of difference are selected, and when the gas test sample is applied to the cell the resulting features are compared with the said standards at the selected optimum points.

13. A method according to any of the preceding claims, in which the derivation of the parameters and the comparison with standard parameters is performed by an electronic computer or microprocessor, using a pre-arranged computer programme.

14. Apparatus for performing the method of any of the preceding claims, comprising an electrochemical fuel cell (7,20), means for delivering a gas sample (23,26,27) thereto, and a computer or microprocessor (14) arranged to receive the voltage output from the cell, and programmed to produce or derive one or more of the features specified, and optionally to compare a feature with a corresponding feature from a previous sample.

Fig. 1.

Fig. 2.

0172969

2/9

FIG.3.

FIG.4.

0172969

3/9

Fig. 5.

Fig. 6.

FIG.7.

FIG. 8.

FIG. 9.

FIG. 10.

Fig.11.

KEY
⊡ TIME VALUES
⊙ POLYNOMIAL COEFFICIENTS (t–t5)
• D.F.T. COEFFICIENTS

6/6

0172969